# EUROPÄISCHE PATENTSCHRIFT

(11) **EP 0 429 587 B1**
(45) Veröffentlichungstag und Bekanntmachung des Hinweises auf die Patenterteilung: **12.04.1995**
(21) Anmeldenummer: 90908201.8
(22) Anmeldetag: 31.05.1990
(51) Int. Cl.: C12P 21/02, C12N 5/10

(54) **VERFAHREN ZUR HERSTELLUNG EINES REGULATIONSPROTEINS (NEF) VON HIV-1 UNTER VERWENDUNG DER HUMANEN ZELLINIE TH4-7-5**
PROCESS FOR OBTAINING A REGULATION PROTEIN (NEF) FOR HIV-1 USING THE HUMAN CELL LINE TH4-7-5
PROCEDE DE PRODUCTION D'UNE PROTEINE DE REGULATION (NEF) DU VIH-1 AU MOYEN DE LA LIGNEE TH4-7-5 DE CELLULES HUMAINES

(30) Priorität: 31.05.1989 DE 3917775; 07.12.1989 DE 3940527
(43) Veröffentlichungstag der Anmeldung: 05.06.1991
(73) Patentinhaber: GSF-Forschungszentrum für Umwelt und Gesundheit, GmbH, 85764 Oberschleissheim (DE)
(72) Erfinder: ERFLE, Volker, D-8000 München 80 (DE); MELLERT, Werner, D-8044 Lohhof (DE)
(74) Vertreter: Klunker . Schmitt-Nilson . Hirsch
(86) Internationale Anmeldenummer: EP9000869
(87) Internationale Veröffentlichungsnummer: WO9015150

(56) Entgegenhaltungen:
- WO-A-87/02988
- ABSTRACTS OF THE 6th INTERNATL. CONFERENCE ON AIDS, University of California, San Francisco, CA (US) 22 June 1990; R. HERMANN et al., no. FA-302#
- JOURNAL OF VIROLOGY, vol. 61, no. 12, December 1987; S. DEWHURST et al., pp. 3774-3782#
- VIROLOGY, vol. 168, 1989; R. ANAND et al., pp. 79-89#

## Beschreibung

Die Erfindung betrifft ein Verfahren zur Herstellung eines Regulationsproteins von HIV-1 unter Verwendung der humanen Zelllinie TH4-7-5.

Das HIV-1-Regulationsprotein "nef" rückt derzeit in den Vordergrund der Aids-Forschung, da es wichtige Aufschlüsse über die Regulation des HIV-Virus gibt, als serologischer Marker für die Früherkennung der Infektion eingesetzt werden kann und möglicherweise an der Entstehung neurologischer Komplikationen beteiligt ist. Um nef-Proteine herzustellen, werden zur Zeit Bakterien, beispielsweise E. coli-Bakterien, mit Expressionsvektoren benutzt. Diese Systeme haben jedoch den Nachteil, daß die entstehenden Proteine nicht myristilisiert sind und somit möglicherweise andere antigene und funktionelle Eigenschaften besitzen können als das in vivo produzierte Protein. Die beste Quelle für die nef-Regulationsproteine wären demzufolge HIV-infizierte humane Zellen, die ein nef-Protein produzieren, das mit dem in vivo produzierten Protein identisch ist. Die Gewinnung von diesen nef-Regulationsproteinen aus HIV-infizierten, humanen Zellen war bislang jedoch nicht möglich, da infizierte T-Zellen kaum nachweisbare Mengen an nef-Regulationsproteinen produzieren.

Aus der WO-A-8 702 988 ist das HIV-1-Regulationsprotein bekannt. Es wird durch Infizierung verschiedener Zellinien mit HIV-T und Cokultivierung hergestellt.

Aus dem Journal of Virology, Vol. 61, no. 12, Dez. 87, S. 3774 - 3782 ist die Infizierung einer humanen Gliazellinie und Klonierung der infizierten Zellen bekannt.

Der Erfindung liegt daher die Aufgabe zugrunde, eine Quelle für die ner-Regulationsproteine in Form von HIV-infizierten humanen Zellen, die ein nef-Protein produzieren, das mit dem in-vivo produzierten Protein identische ist, zur Verfügung zu stellen.

Erfindungsgemäß wird vorgeschlagen, die humane Zellinie TH4-7-5 zur Herstellung des HIV-1-Regulationsproteins "nef" zu verwenden. Die Zellen werden mit HIV infiziert, kloniert und das HIV-1-Regulationsprotein"nef" daraus isoliert.

Die humane Zellinie TH4-7-5 stammt aus einem humanen Astrozytom. Die ursprüngliche Zellinie 85HG-66 (Stavrou et al, J. Neurol. Sciences 80, 205-220, 1987) wurde mit HIV-1 (HTLV-IIIB) infiziert und kloniert. Der daraus resultierende HIV-positive Klon wurde TH4-7-5 genannt.

Die erfindungsgemäße humane Zellinie TH4-7-5 wurde am 30. Mai 1989 bei der Collection Nationale de Cultures des Microorganismes (CNCM), Paris, unter der Hinterlegungsnummer I-861 hinterlegt.

Die erfindungsgemäße humane Zellinie TH4-7-5 wächst als Monolayer-Kultur bei 37°C und 5% CO₂ in RPMI 1640 (Gibco) als Kulturmedium, das mit 10% fetalem Kälberserum versetzt ist. Die Morphologie der Zellen ist fibroblastenartig. Die Zellen exprimieren typische Merkmale von Gliazellen wie Glial Fibrillary Acidic Protein (GFAP) sowie S100-Protein.

Die Erfindung wird nachfolgend unter Bezugnahme auf die Zeichnungen näher erläutert. In den Zeichnungen zeigt:
- Fig. 1: eine mikroskopische Darstellung der TH4-7-5 Zellen nach Infektion und Anfärbung der HIV-positiven Zellen mit Antiseren, die Strukturproteine von HIV-1 erkennen;
- Fig. 2: eine mikroskopische Darstellung eines Subklons von TH4-7-5-Zellen nach Anfärbung der "nef"-positiven Zellen mit einem Antiserum, das das nef-Protein erkennt; und
- Fig. 3: einen Immunblot ("Western Blot") eines TH4-7-5-Zellextrakts unter Verwendung eines Antiserums gegen "nef".

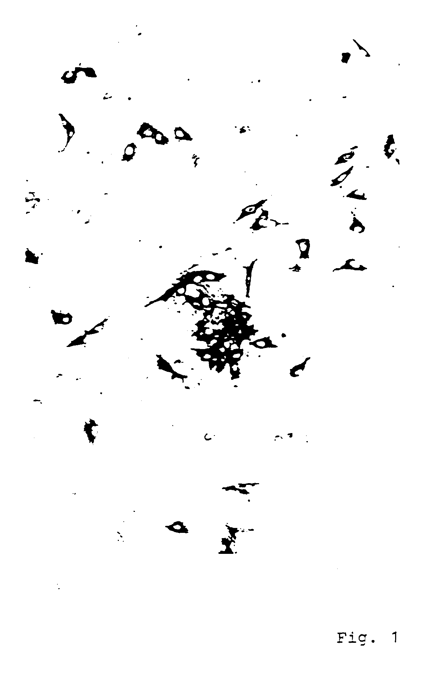

Zur Verwendung der humanen Zellinie TH4-7-5 für die Produktion von "nef" wurde die Ursprungslinie 85HG-66 zunächst mit HIV-1 (HTLV-IIIB) infiziert. Dies führte etwa 1-2 Wochen nach der Infektion zu einer Zellkultur, bei der etwa 1% der Zellen infiziert waren, die HIV-Strukturproteine produzierten. Der Nachweis der HIV-Strukturproteine erfolgte mit einem serologischen Test unter Verwendung von Antikörpern gegen diese Proteine (erster Antikörper) und mit einem gegen das entsprechende Immunglobulin gerichteten Antikörper (zweiter Antikörper), an den Meerrettichperoxidase gekoppelt ist. Zur Sichtbarmachung des Antigen-Antikörperkomplexes wurde als spezifisches Substrat 3-Aminoethylcarbazol verwendet, das wasserunlöslich ist. Dieses Verfahren ist bekannt als sogenannte indirekte Immunperoxidasefärbung; vgl. dazu Mellert et al, A1FO1, 105-107, 1987. Die Anzahl der gefärbten Zellen wurde mit Hilfe eines Lichtmikroskopes bestimmt. Die Fig. 1 zeigt die mit HIV-1 infizierten Zellen der humanen Zellinie TH4-7-5, die nach diesen Färbeverfahren angefärbt worden sind.

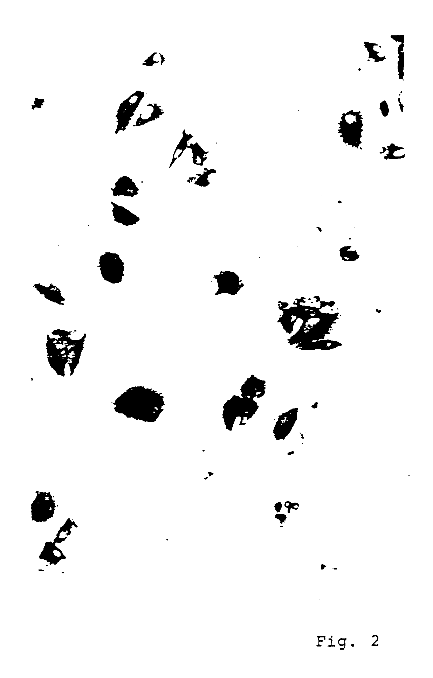

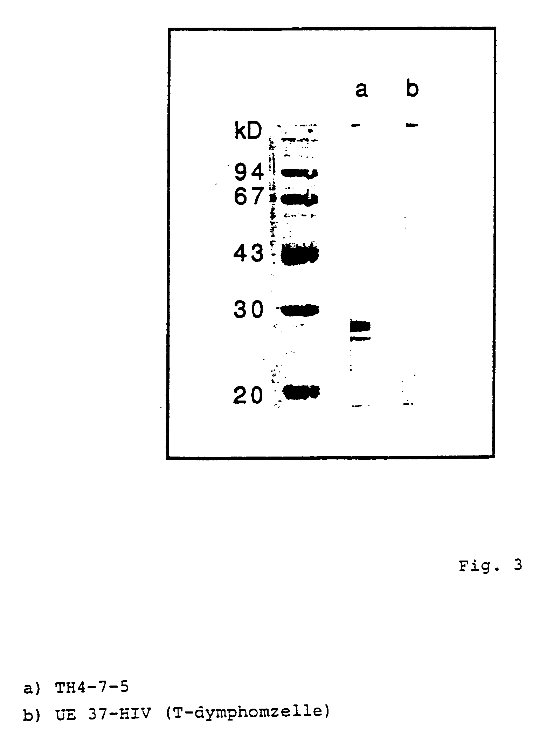

Durch Klonierung der infizierten humanen Zellinie TH4-7-5 wurden Subklone erhalten, bei denen der Prozentsatz an HIV-positiven Zellen bis zu 100% betrug. Diese Subklone wurden daraufhin auf die Expression von "nef"-Protein untersucht. Dabei wurde ein Antiserum verwendet, das das "nef"-Protein erkennt. Diese Untersuchungen wurden mit einem polyklonalen Antikörper von Kaninchen gegen "nef" unter Zuhilfenahme der indirekten Immunperoxidasefärbung sowie des Immunblots (Western Blot, vgl. dazu Erfle und Mellert, Methods of Encymatic Analysis 10, 469-483, 1986) durchgeführt. Die Untersuchungsergebnisse unter Verwendung der indirekten Immunperoxidasetechnik sind in Fig. 2 und die Untersuchungsergebnisse unter Zuhilfenahme des Immunblots in Fig. 3 dargestellt.

Beide Untersuchungsmethoden ergaben, daß die TH4-7-5 Zellinie beträchtliche Mengen an "nef"-Protein produzieren. Im Immunblot, dessen Ergebnisse in Fig. 3 dargestellt sind, ist erkennbar, daß von den Zellen ein "nef"-Protein mit dem erwarteten Molekulargewicht von 27.000 Dalton produziert wird.

## Patentansprüche

1. Humane Zellinie TH4-7-5, hinterlegt bei der Collection Nationale de Cultures des Microorganismes (CNCM), Paris, unter der Hinterlegungsnummber I-861.

2. Verwendung der humanen Zellinie TH4-7-5 gemäß Anspruch 1 zur Produktion von HIV-1-Regulationsprotein.

## Claims

1. A human cell line TH4-7-5, deposited under deposit number I-861 at the Collection Nationale des Cultures des Microorganismes (CNCM), Paris.

2. Use of the human cell line TH4-7-5 of claim 1 for producing HIV-1 regulation protein.

## Revendications

1. Lignée cellulaire humaine TH4-7-5, déposée auprès de la Collection Nationale de culture des microorganismes (CNCM) à Paris sous le numéro de dépôt I-861.

2. Application de la lignée cellulaire humaine TH4-7-5 selon la revendication 1 pour la production de protéines de régulation de VIH-1.
